(19) Europäisches Patentamt
European Patent Office
Office européen des brevets

(11) Publication number: **0 486 702 A1**

(12) **EUROPEAN PATENT APPLICATION published in accordance with Art. 158(3) EPC**

(21) Application number: **91910649.2**

(51) Int. Cl.5: **C07C 279/18**

(22) Date of filing: **07.06.91**

(86) International application number: **PCT/JP91/00773**

(87) International publication number: **WO 91/18869 (12.12.91 91/28)**

(30) Priority: **08.06.90 JP 148674/90**

(43) Date of publication of application: **27.05.92 Bulletin 92/22**

(84) Designated Contracting States: **CH DE ES FR GB IT LI**

(71) Applicant: **Asahi Kasei Kogyo Kabushiki Kaisha**
**2-6, Dojimahama 1-chome Kita-ku**
**Osaka-shi Osaka 530(JP)**

(72) Inventor: **HAYASHI, Yoshiharu**
**1-4-22-708, Teramae, Kanazawa-ku**
**Yokohama-shi, Kanagawa 236(JP)**
Inventor: **YAMAURA, Yuji**
**Room 771, Minami-Apt., 5-1, Midorigaoka 4-chome**
**Nobeoka-shi, Miyazaki 882(JP)**

(74) Representative: **Blake, John Henry Francis et al**
**BROOKES AND MARTIN High Holborn House**
**52/54 High Holborn**
**London WC1V 6SE(GB)**

# (54) PHENYL 4-GUANIDINOBENZOATE DERIVATIVE, PROCESS FOR PRODUCING THE SAME, AND PROTEASE INHIBITOR CONTAINING THE SAME.

(57) A phenyl 4-guanidinobenzoate derivative of general formula (I) and a pharmacologically acceptable acid addition salt thereof, each having an excellent protease inhibitor activity, wherein $R^1$ represents hydrogen or halogen; $R^2$ represents trifluoromethoxy, $OCOR^3$, $C(=NOR^4)R^5$ or styrenesulfonyl; $R^3$ represents (un)substituted $C_3$ to $C_7$ cycloalkyl; $R^4$ represents hydrogen, $C_1$ to $C_4$ alkyl, $C_1$ to $C_4$ alkenyl or $CH_2COOR^6$; $R^5$ represents hydrogen or $C_1$ to $C_4$ alkyl; and $R^6$ represents hydrogen or $C_1$ to $C_4$ alkyl.

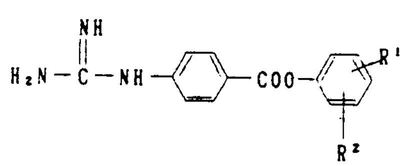

( I )

EP 0 486 702 A1

## FIELD OF THE INVENTION

This invention relates to novel 4-guanidinobenzoic acid phenyl ester derivatives, more particularly 4-guanidinobenzoic acid phenyl ester derivatives having activities to inhibit proteolytic enzymes or pharmacologically acceptable acid addition salts thereof, to a producing process thereof and to proteolytic enzyme inhibitors containing the same as active ingredients.

## BACKGROUND OF THE INVENTION

Pancreatitis is classified into acute pancreatitis and chronic pancreatitis from clinical point. In the case of acute pancreatitis, the pancreas becomes normal both clinically and biologically when the cause or the factor of the disease is removed. In the case of chronic pancreatitis, on the contrary, it is considered that histological or functional changes remain even when the cause or the factor of the disease is removed. With regard to the etiology of pancreatitis, it is said that, though not so clear, alcoholic cause is most frequent in Japan, followed by unknown origin (idiopathic) and cholelith.

Processes of the onset of pancreatitis and subsequent development of the disease (disseminated intravascular coagulation, multi-organ disturbance and the like) are complex and therefore have not been revealed yet to the full. As medical therapy of the disease, anti-proteolytic enzyme agents are being used widely. Typical examples of such compounds are disclosed for instance in JP-A-51-138642, JP-A-57-53454, JP-A-62-155253 and JP-A-63-165357, though their effects are not sufficient (the term "JP-A" as used herein means an "unexamined published Japanese patent application"). Under such circumstances, development has been desired, of more superior proteolytic enzyme inhibitors which are effective even in a complex exacerbation process and have more high safety.

## DISCLOSURE OF THE INVENTION

Principal objects of this invention are to develop materials having proteolytic enzyme inhibiting effects which are superior to those of the prior art anti-proteolytic enzyme agents, a process for producing these materials, and proteolytic enzyme inhibitors which contain these materials as active ingredients and are useful as various drugs.

The inventors of the present invention have conducted intensive studies with the aim of overcoming the aforementioned problems involved in the prior art and found that novel 4-guanidinobenzoic acid phenyl ester derivatives represented by the following general formula (I) are possessed of strong activities to inhibit a broad spectrum of serine proteases such as trypsin, thrombin, plasmin, kallikrein, factor Xa, elastase and the like. The present invention has been accomplished as a result of these efforts.

That is, according to the present invention, there is provided a 4-guanidinobenzoic acid phenyl ester derivative or its pharmacologically acceptable acid addition salt and a proteolytic enzyme inhibitor containing the same as an active ingredient, said derivative represented by the following general formula (I):

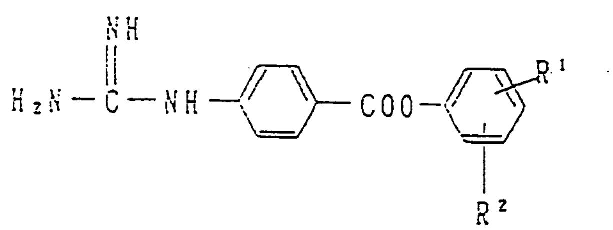

(I)

wherein $R^1$ is a hydrogen atom or a halogen atom; $R^2$ is a trifluoromethoxy group, $OCOR^3$, $C(=NOR^4)R^5$ or a styrene sulfonyl group; $R^3$ is a substituted or un-substituted $C_{3-7}$ cycloalkyl group; $R^4$ is a hydrogen atom, a $C_{1-4}$ alkyl group, a $C_{1-4}$ alkenyl group or $CH_2COOR^6$; $R^5$ is a hydrogen atom or a $C_{1-4}$ alkyl group; and $R^6$ is a hydrogen atom or a $C_{1-4}$ alkyl group.

Illustrative examples of the pharmacologically acceptable acid addition salt include: inorganic acid addition salts such as a hydrochloride, hydrobromide, hydroiodide, sulfate, perchlorate, phosphate, nitrate and the like; and organic acid addition salts such as acetate, oxalate, maleate, fumarate, succinate, methane sulfonate, ethane sulfonate, toluene sulfonate and the like.

The inventive compound represented by the general formula (I) may be obtained for example by allowing 4-guanidinobenzoic acid represented by the following general formula (II) or its reactive derivative

to perform esterification reaction with a compound represented by the following general formula (III);

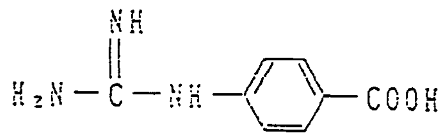

(II)

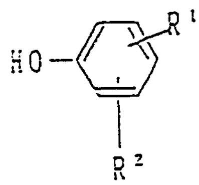

(III)

wherein $R^1$ and $R^2$ are the same as those described in the foregoing.

More particularly, the compound of the present invention as represented by the general formula (I) can be produced by reacting an acid halide of the 4-guanidinobenzoic acid represented by the general formula (II), i.e., a compound represented by the following general formula (IV), which can be obtained easily by heating the compound (II) together with thionyl chloride, with the compound of the general formula (III) in the presence of an acid bonding agent such as pyridine, triethylamine or the like;

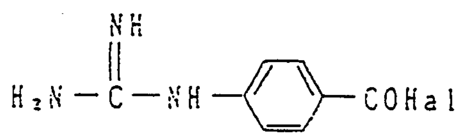

(IV)

wherein Hal is a halogen atom such as chlorine, bromine, iodine or the like.

Solvent to be used in such a reaction system may be selected from aqueous media or organic solvents including for instance: ether solvents such as ether, tetrahydrofuran and the like; aromatic solvents such as benzene, toluene and the like; heterocyclic solvents such as pyridine and the like; and improtonic polar solvents such as N,N-dimethylformamide, N,N-dimethylacetamide, dimethylsulfoxide and the like, of which pyridine is particularly preferred. When solvent other than pyridine is used, an acid bonding agent may be used jointly including organic bases such as triethylamine, tributylamine, dimethylamine, pyridine and the like and inorganic bases such as calcium carbonate, sodium carbonate, sodium hydroxide and the like.

An alternative method may comprise allowing 4-guanidinobenzoic acid represented by the general formula (II) to perform direct condensation with the compound of the general formula (III) without using any solvent or in an organic solvent in the presence of a dehydrating agent such as DCC (1,3-dicyclohexylcarbodiimide), EDC [1-ethyl-3-(3-dimethylaminopropyl)carbodiimide hydrochloride] or the like.

The inventive compound of the general formula (I) prepared by any of these processes may be derived into pharmacologically acceptable acid addition salts in the usual way.

For example, the compound of the general formula (I) thus obtained can be made into a carbonate by treating the compound with an aqueous solution of sodium hydrogencarbonate and, if desired, the resulting carbonate of the compound can easily be derived further into inorganic acid salts such as hydrochloride, hydrobromide, hydroiodide, sulfate, perchlorate, phosphate, nitrate and the like or into organic acid salts such as acetate, oxalate, maleate, fumarate, succinate, methane sulfonate, ethane sulfonate, toluene sulfonate and the like.

Illustrative examples of the compound of the present invention are listed below to make description of the present invention easy. As a matter of course, these illustrative compounds do not limit the scope of the present invention.

(1) 2-trifluoromethoxyphenyl 4-guanidinobenzoate
(2) 3-trifluoromethoxyphenyl 4-guanidinobenzoate
(3) 4-trifluoromethoxyphenyl 4-guanidinobenzoate
(4) 3-cyclopropylcarbonyloxyphenyl 4-guanidinobenzoate
(5) 3-(1-methylcyclopropylcarbonyloxy)phenyl 4-guanidinobenzoate

(6) 3-cyclobutylcarbonyloxyphenyl 4-guanidinobenzoate
(7) 3-cyclopentylcarbonyloxyphenyl 4-guanidinobenzoate
(8) 3-cyclohexylcarbonyloxyphenyl 4-guanidinobenzoate
(9) 3-(1-hydroxyiminoethyl)phenyl 4-guanidinobenzoate
(10) 3-(1-methoxyiminoethyl)phenyl 4-guanidinobenzoate
(11) 3-(1-allyloxyiminoethyl)phenyl 4-guanidinobenzoate
(12) 3-(1-carboxylmethoxyiminoethyl)phenyl 4-guanidinobenzoate
(13) 3-(1-methoxycarbonylmethoxyiminoethyl)phenyl 4-guanidinobenzoate
(14) 3-(1-ethoxycarbonylmethoxyiminoethyl)phenyl 4-guanidinobenzoate
(15) 3-hydroxyiminomethylphenyl 4-guanidinobenzoate
(16) 3-methoxyiminomethylphenyl 4-guanidinobenzoate
(17) 3-ethoxyiminomethylphenyl 4-guanidinobenzoate
(18) 3-methoxycarbonylmethoxyiminomethylphenyl 4-guanidinobenzoate
(19) 3-ethoxycarbonylmethoxyiminomethylphenyl 4-guanidinobenzoate
(20) 2-($\beta$-styrenesulfonyloxy)phenyl 4-guanidinobenzoate
(21) 3-($\beta$-styrenesulfonyloxy)phenyl 4-guanidinobenzoate
(22) 4-($\beta$-styrenesulfonyloxy)phenyl 4-guanidinobenzoate
(23) 2-chloro-5-cyclopropylcarbonyloxyphenyl 4-guanidinobenzoate
(24) 3-chloro-5-cyclopropylcarbonyloxyphenyl 4-guanidinobenzoate
(25) 2-chloro-5-(1-methylcyclopropylcarbonyloxy)phenyl 4-guanidinobenzoate
(26) 3-chloro-5-(1-methylcyclopropylcarbonyloxy)phenyl 4-guanidinobenzoate
(27) 3-bromo-5-(1-methylcyclopropylcarbonyloxy)phenyl 4-guanidinobenzoate
(28) 2-fluoro-5-(1-methylcyclopropylcarbonyloxy)phenyl 4-guanidinobenzoate
(29) 3-chloro-5-cyclobutylcarbonyloxyphenyl 4-guanidinobenzoate
(30) 3-chloro-5-cyclopentylcarbonyloxyphenyl 4-guanidinobenzoate
(31) 3-chloro-5-cyclohexylcarbonyloxyphenyl 4-guanidinobenzoate
(32) 2-chloro-5-(1-hydroxyiminoethyl)phenyl 4-guanidinobenzoate
(33) 2-chloro-5-(1-methoxyiminoethyl)phenyl 4-guanidinobenzoate
(34) 2-chloro-5-(1-methoxycarbonylmethoxyiminoethyl)phenyl 4-guanidinobenzoate
(35) 2-chloro-3-($\beta$-styrenesulfonyloxy)phenyl 4-guanidinobenzoate
(36) 2-chloro-4-($\beta$-styrenesulfonyloxy)phenyl 4-guanidinobenzoate
(37) 2-chloro-5-($\beta$-styrenesulfonyloxy)phenyl 4-guanidinobenzoate
(38) 3-chloro-4-($\beta$-styrenesulfonyloxy)phenyl 4-guanidinobenzoate
(39) 3-chloro-5-($\beta$-styrenesulfonyloxy)phenyl 4-guanidinobenzoate
(40) 3-chloro-6-($\beta$-styrenesulfonyloxy)phenyl 4-guanidinobenzoate

Since these compounds represented by the general formula (I) and their pharmacologically acceptable acid addition salts are possessed of markedly strong functions to inhibit activities of enzymes such as trypsin, thrombin, plasmin, factor Xa, elastase and the like, these compounds and their salts find versatile use in such applications as: therapeutic agents of acute and chronic pancreatitis based on their trypsin inhibiting activity; therapeutic agents of hemorrhagic diseases and thrombosis based on their functions to inhibit activities of thrombin, plasmin and factor Xa; and treatment and/or prevention of diseases caused by abnormal accentuation of decomposition of protein such as elastin on the basis of their function to inhibit elastase activity.

When the compound of the present invention is applied as therapeutic agent to suffers from these diseases, dose of the agent may vary depending on the kind of the disease, degree of the symptom, age, health state and body weight of each patient, kind of other drug when used simultaneously, frequency of the treatment, expected therapeutic effect and the like. Though not strictly limited because of these variable factors, the agent prepared from the compound of the present invention may generally be administered orally or parenterally in a dose of from about 5 to about 1,000 mg, preferably from about 10 to about 500 mg, per day per adult once a day or several times a day by diving the daily dose.

The inventive therapeutic agent may be made into various dosage forms such as powders, fine subtilaes, granules, tablets, capsules, suppositories, injections and the like. These dosage forms may be prepared in the usual way using usually used preparation carriers.

Fillers to be used in the pharmaceutical preparation include for instance lactose, corn starch, sucrose, glucose, sorbitol, crystalline cellulose, silicon dioxide and the like. Useful binders include for example polyvinyl alcohol, polyvinyl ether, ethyl cellulose, methyl cellulose, gum arabic, traganth, gelatin, shellac, hydroxypropyl cellulose, hydroxypropyl starch, polyvinyl pyrrolidone and the like. Useful disintegrating agents include for example starch, agar, gelatin powder, crystalline cellulose, calcium carbonate, sodium

hydrogencarbonate, calcium citrate, fibrin calcium gluconate, dextrin, pectin and the like. As for lubricants to be used in the pharmaceutical preparation, magnesium stearate, talc, polyethylene glycol, silica, hydrogenated plant oil and the like are useful. Coloring agents authorized to be added to drugs may also be used in the therapeutic agent of the present invention. In addition, cocoa powder, peppermint oil, aromatic acid, cinnamon powder and the like may be used as correctives. As a matter of course, an appropriate coating such as sugar coating, gelatin coating or the like may be applied when the inventive therapeutic agent is made into tablets or granules.

In the case of the preparation of injections, intravenous injections may be prepared in the usual way by, if necessary, adding to the principal agent a pH adjusting agent, a buffer, a stabilizer, a solubilizing agent and the like.

## BEST MODE FOR THE PRACTICE OF THE INVENTION

Examples of the present invention are given below by way of illustration and not by way of limitation.

## EXAMPLE 1

Synthesis of 3-chloro-5-($\beta$-styrenesulfonyloxy)phenyl 4-guanidinobenzoate methanesulfonic acid salt (Compound 39):

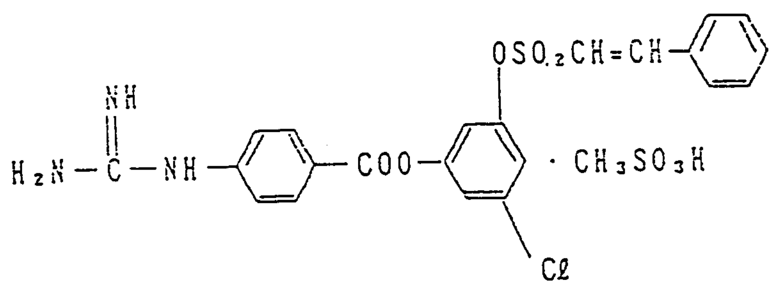

(V)

A mixture consisting of 1.2 g of 4-guanidinobenzoylchloride hydrochloride, 1.6 g of 3-chloro-5-($\beta$-styrenesulfonyloxy)phenoland 25 ml of pyridine was stirred for 30 minutes in an ice bath. After completion of the reaction, pyridine was distilled off under a reduced pressure and the remaining reaction product was dissolved in water. The resulting solution was then mixed with a saturated aqueous solution of sodium hydrogencarbonate to obtain carbonic acid salt of the title compound (V). The compound thus obtained in a crystalline form was washed with water and acetone in that order and then dried in vacuo. The crystals were suspended in methanol and the suspension was mixed with 0.25 g of methanesulfonic acid. Thereafter, the thus prepared mixture was diluted with diethyl ether and the resulting crystals were dried in vacuo to obtain 1.1 g of the title compound (V) in a white crystalline form.

- Nuclear magnetic resonance spectrum (DMSO-$d_6$; ppm):
  2.42(s,3H), 7.46 - 8.17(m,18H), 10.19(s,1H)
- Infrared adsorption spectrum (KBr pellet; $cm^{-1}$)
  1734.2 1684.1 1595.2 1570.1 1437.5 1374.9 1249.0 1211.8 1172.1 1117.4 1069.2 1047.9 976.0 884.0 824.8 757.6
- Elementary analysis

| | | | | | |
|---|---|---|---|---|---|
| Theoretical value (%); | C: 48.63, | H: 3.90, | Cl: 6.24 | N: 7.39, | S: 11.28 |
| Found value (%); | C: 48.98, | H: 3.79, | Cl: 6.10 | N: 7.45, | S: 11.43 |

As shown in the following reaction formula, compounds of the present invention (Compound 3 and Compound 2) were prepared by repeating the above process except that 3-chloro-5-($\beta$-styrenesulfonyloxy)-phenol was replaced by respective phenol derivatives, with the results shown in Table 1.

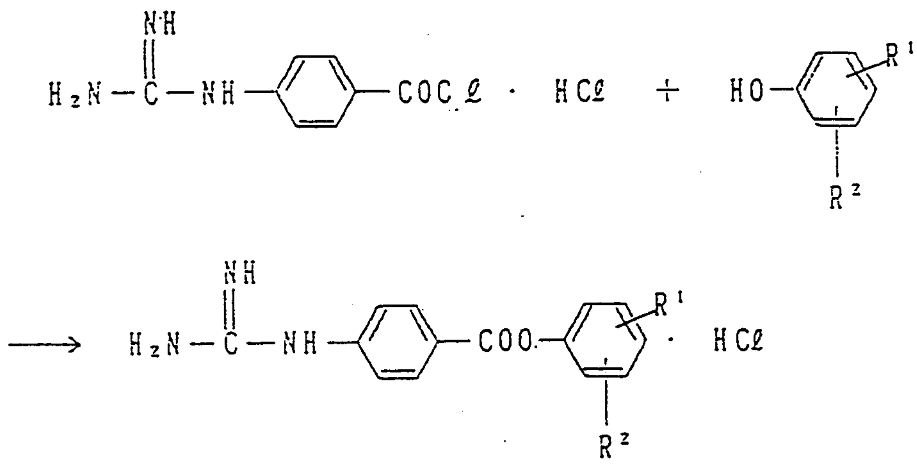

(In this formula, $R^1$ is H and $R^2$ is 4-trifluoromethoxy or 3-trifluoromethoxy)

TABLE 1 PHYSICAL PROPERTIES

| Compound No. | $R^1$ | $R^2$ | Type of Salt | Hydrogen Nuclear Magnetic Resonance Spectrum ($DMSO-d_6$; ppm) ........ Infrared Adsorption Spectrum (KBr; $cm^{-1}$) | Elementary Analysis |
|---|---|---|---|---|---|
| 3 | H | 4-trifluoro-methoxy | mesyl acid salt | 2.39 (s,3H)<br>7.43 - 8.20(m,12H)<br>10.14(s,1H)<br>........<br>1734.1, 1684.0, 1608,0. 1570.0, 1506.1, 1272.2, 1186.6, 1071.9, 1048.0, 1015.9, 885.4, 784.7, 764.2 | Theoretical Value (%)<br>C: 44.14, H: 3,70,<br>N: 9.65, S: 7.36<br>Found Value (%)<br>C: 44.55, H: 3.61,<br>N: 9.49, S: 7.47 |
| 2 | H | 3-trifluoro-methoxy | mesyl acid salt | 2.47(s,3H)<br>7.36 - 8.19(m,12H)<br>10.28(s,1H)<br>........<br>1734.1, 1684.0, 1489.9, 1260.3, 1047.6, 870.9, 781.9, 759.5, 631.1, 532.2 | Theoretical Value (%)<br>C: 44.14, H: 3.70,<br>N: 9.65, S: 7.36<br>Found Value (%)<br>C: 44.47, H: 3.54,<br>N: 9.52, S: 7.53 |

EXAMPLE 2

Synthesis of 3-chloro-5-(1-methylcyclopropylcarbonyloxy)phenyl 4-guanidinobenzoate methanesulfonic acid salt (Compound 26):

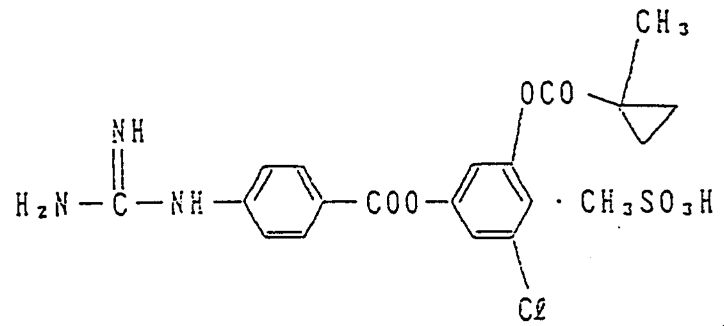

(VI)

A 1.1 g portion of 4-guanidinobenzoic acid hydrochloric acid salt was dissolved in 10 ml of a 1:1 v/v mixture of anhydrous pyridine and DMF (N,N-dimethylformamide). After cooling down to -20 ° C, the solution was mixed with 1.1 g of DCC (N,N-dicyclohexylcarbodiimide) and stirred for 15 minutes. The resulting solution was further mixed with 1.2 g of 3-chloro-5-(1-methylcyclopropylcarbonyloxy)phenol and stirred for 24 hours with gradual increase in the reaction temperature to room temperature. After completion of the reaction, solvents were distilled off under a reduced pressure and water was added to the remaining contents. The resulting solution, after removing insoluble materials by filtration, was then mixed with a saturated aqueous soltuion of sodium hydrogencarbonate to obtain carbonic acid salt of the title compound (VI). The compound thus obtained in a crystalline form was washed with water and acetone in that order and then dried in vacuo. The crystals were suspended in methanol and the suspension was mixed with 0.2 g of methanesulfonic acid. Thereafter, the thus prepared mixture was diluted with diethyl ether and the resulting crystals were dried in vacuo to obtain 1.0 g of the title compound (VI) in a white crystalline form.

- Nuclear magnetic resonance spectrum (DMSO-$d_6$; ppm):
  0.90 - 0.97(m,2H), 1.32 - 1.38(m,5H), 2.38(s,3H), 7.21 - 8.17(m,11H), 10.29(s,1H)
- Infrared adsorption spectrum (KBr pellet; $cm^{-1}$)
  1734.3 1684.4 1628.3 1599.4 1570.0 1447.6 1322.3 1268.6 1183.2 1134.0 1074.7 1048.5 848.7 785.2 668.0 553.9
- Elementary analysis

| | | | | | |
|---|---|---|---|---|---|
| Theoretical value (%); | C: 49.64, | H: 4.58, | Cl: 7.32 | N: 8.68, | S: 6.62 |
| Found value (%); | C: 49.85, | H: 4.47, | Cl: 7.18 | N: 8.50, | S: 6.75 |

As shown in the following reaction formula, compounds of the present invention (Compound 31 and Compound 10) were prepared by repeating the above process except that 3-chloro-5-(1-methylcyclopropylcarbonyloxy)phenol was replaced by respective phenol derivatives, with the results shown in Table 2.

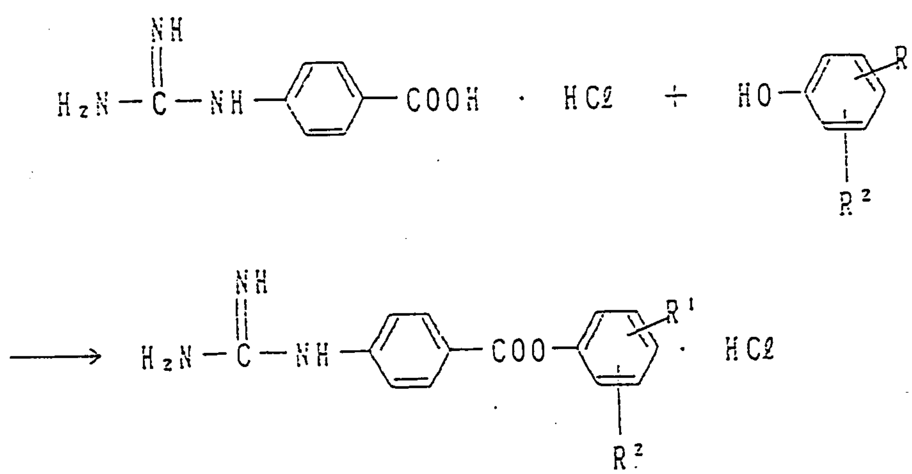

[In this formula, $R^1$ is 3-Cl or H and $R^2$ is 5-cyclohexylcarbonyloxy or 3-(1 methoxyiminoethyl)]

TABLE 2 PHYSICAL PROPERTIES

| Compound No. | $R^1$ | $R^2$ | Type of Salt | Hydrogen Nuclear Magnetic Resonance Spectrum ($DMSO-d_6$; ppm) ........ Infrared Adsorption Spectrum (KBr; $cm^{-1}$) | Elementary Analysis |
|---|---|---|---|---|---|
| 31 | 3-Cl | 5-(cyclohexyl carbonyloxy) | mesyl acid salt | 1.22 - 1.79(m,11H), 2.25(s,3H)<br>7.08 - 8.04(m,11H)<br>10.50(s,1H)<br>........<br>1740.2, 1684.3, 1654.1, 1636.3, 1597.1, 1576.1, 1252.7, 1209.8, 1131.7, 1070.4, 1044.8, 1018.4, 761.6, 669.9 | Theoretical Value (%)<br>C: 51.61, H: 5.11,<br>Cl: 6.92, N: 8.20,<br>S: 6.26<br>Found Value (%)<br>C: 51.55, H: 5.34,<br>Cl: 7.18, N: 8.11,<br>S: 6.01 |
| 10 | H | 3-(1-methoxy iminoethyl) | mesyl acid salt | 2.20(s,3H), 2.41(s,3H),<br>3.93(s,3H), 7.32 - 8.19(m,12H)<br>10.17(s,1H)<br>........<br>1733.9, 1684.1, 1636.1, 1610.9, 1575.1, 1508.2, 1308.5, 1261.8, 1196.6, 1077.3, 1047.9, 914.4, 867.7, 782.1 | Theoretical Value (%)<br>C: 51,17, H: 5.24,<br>N: 13,26, S: 7.58<br>Found Value (%)<br>C: 51.46, H: 5.19,<br>N: 13.22, S: 7.73 |

PREPARATION EXAMPLE 1

A total of 100 tablets, each containing 100 mg of active ingredient, were prepared by making them in

the usual way from a mixture consisting of 10 g of 3-chloro-5-(β-styrenesulfonyloxy) 4-guanidinobenzoic acid phenyl ester methanesulfonic acid salt (Compound 39), 400 mg of fibrin calcium gluconate (disintegrating agent), 200 mg of magnesium stearate (lubricant) and 9.4 g of fine crystalline cellulose.

PREPARATION EXAMPLE 2

In 10 ml of ethanol was dissolved 1 g of 3-chloro-5-(β-styrenesulfonyloxy) 4-guanidinobenzoic acid phenyl ester methanesulfonic acid salt (Compound 39). After sterilizing through a bacterial filter, a 0.5 ml portion of the sterile solution was included in a 5 ml capacity ampule (50 mg active ingredient per ampule) followed by sealing them. The content in the thus prepared ampule is used as an injection after diluting it to a volume of 2.5 ml with an appropriate amount of a diluent such as Tris-HCl buffer (pH 8.6).

The following examples of experiments are provided to describe pharmacological functions of the compound of the present invention.

PHARMACOLOGICAL TEST EXAMPLE 1

The method disclosed in JP-A-2-101017 was slightly modified and used for the measurement of the function of typical compounds belonging to the compound (I) of the present invention (Compounds 39, 3, 2, 26, 31 and 10) in terms of their in vitro activities to inhibit trypsin, thrombin, plasmin, factor Xa and elastase.

Substrate used for the enzyme reaction were: benzoyl-Arg-7-amido-4-methylcoumalin for trypsin, Boc-Val-Pro-Arg-7-amido-4-methylcoumalin for thrombin, Boc-Val-Leu-Lys-7-amido-4-methylcoumalin for plasmin, Boc-Ile-Glu-Gly-Arg-7-amido-4-methylcoumalin for factor Xa and methoxysuccinyl-Ala-Ala-Pro-Val-7-amido-4-methylcoumalin for elastase. Using these substrate, the 50% inhibition concentration ($IC_{50}$) was measured. Each enzyme reaction was carried out in 0.2 M Tris-HCl buffer supplemented with 0.02 M $CaCl_2$ and 0.005% Triton X-100 at pH 8.0 for trypsin, thrombin, plasmin and factor Xa or at pH 7.2 for elastase. Each sample solution to be measured was mixed with each enzyme solution and the reaction mixture was incubated at 25° C for 60 minutes in the case of trypsin or for 20 minutes in other cases of enzymes. In this instance, a compound disclosed in JP-A-51-138642 was used as a Comparative Example 1, and a compound disclosed in JP-A-63-165357 as a Comparative Example 2. The results are shown in Table 3.

TABLE 3 RESULT OF EVALUATION

| Compound No. | 50% Inhibition Concentration ($IC_{50}$) (M) | | | | |
|---|---|---|---|---|---|
| | Trypsin | Thrombin | Plasmin | Factor Xa | Elastase |
| 39 | 3.6 x $10^{-10}$ | 2.1 x $10^{-8}$ | 1.2 x $10^{-8}$ | 1.6 x $10^{-7}$ | 2.0 x $10^{-9}$ |
| 3 | 8.4 x $10^{-10}$ | 3.9 x $10^{-8}$ | 1.8 x $10^{-8}$ | 4.4 x $10^{-7}$ | 8.9 x $10^{-7}$ |
| 2 | 1.7 x $10^{-10}$ | 7.7 x $10^{-8}$ | 4.3 x $10^{-8}$ | 4.3 x $10^{-7}$ | 2.4 x $10^{-7}$ |
| 26 | 2.5 x $10^{-10}$ | 6.9 x $10^{-8}$ | 1.5 x $10^{-8}$ | 3.4 x $10^{-7}$ | 1.4 x $10^{-8}$ |
| 31 | 4.7 x $10^{-10}$ | 5.8 x $10^{-7}$ | 1.0 x $10^{-8}$ | 3.6 x $10^{-6}$ | 2.3 x $10^{-8}$ |
| 10 | 5.2 x $10^{-10}$ | 1.4 x $10^{-7}$ | 6.3 x $10^{-8}$ | 2.5 x $10^{-6}$ | 4.8 x $10^{-6}$ |
| Comp. Ex. 1*1 | 2.5 x $10^{-8}$ | 5.1 x $10^{-6}$ | 1.2 x $10^{-7}$ | 5.6 x $10^{-5}$ | 1.4 x $10^{-3}$ |
| Comp. Ex. 2*2 | 9.5 x $10^{-9}$ | 8.8 x $10^{-7}$ | 9.0 x $10^{-8}$ | 8.5 x $10^{-6}$ | 9.0 x $10^{-7}$ |

*1: Comparative Example 1: a compound represented by the following general formula which is disclosed in Japanese Patent Application Kokai No. 51-138642.

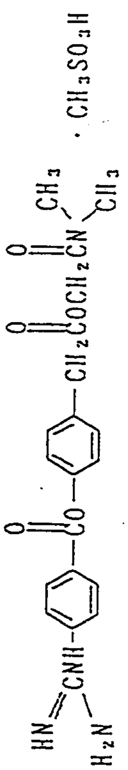

*2: Comparative Example 2: a compound represented by the following general formula which is disclosed in Japanese Patent Application Kokai No. 63-165357.

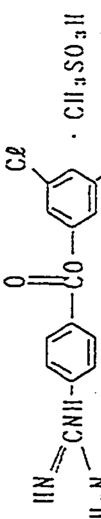

On the basis of these experimental results, it was confirmed that the compound of the present invention has excellent and strong function to inhibit activities of enzymes such as trypsin, thrombin, plasmin, factor Xa and elastase.

In addition, when the inventive compounds used in the above inhibition experiments were subjected to actuate toxicity tests by means of intravenous injection using mice, the $LD_{50}$ value of these compounds was found to be ranging from 50 to 200 mg/kg, thus showing sufficiently low toxicity of the compound of the present invention. It was confirmed therefore that the inventive compound can be used safely as drugs.

PHAMACOLOGICAL TEST EXAMPLE 2

In order to evaluate in vivo drag effects of compound 39 as a typical example of the compound (I) of the present invention, acute pancreatitis edema was induced in rats by intraperitoneal administration of caerulein (40 µg/kg) twice at one hour interval, and the effect of the compound to prevent pancreatitis was examined by oral administration of the compound.

In this instance, male rats of Wistar line (body weight, 300 to 350 g) were used as experimental animals and three of them were made into one group. Experiments were started at eight o'clock in the morning, with the animals in each group under satiation conditions. After measuring their body weights, caerulein pancreatitis was prepared by intraperitoneal administration of caerulein (mfd. by Kyowa Hakko K.K.) (40 µg/kg) twice at one hour interval. Six hours after the second administration, each rat was subjected to laparotomy to collect blood from the abdominal aorta and to excise the pancreas. A polyvinyl pyrrolidone formulation of Compound 39 was prepared (20% by weight of the inventive compound and 80% by weight of polyvinyl pyrrolidone) and orally administered as an aqueous solution (10 mg/ml) 15 minutes before the commencement of the caerulein administration.

Wet weight of the excised pancreas was measured and its ratio to the body weight was calculated. As a comparative example, the same process was repeated except that the inventive compound was replaced by the compound used in Pharmacological Trial 1 as Comparative Example 1, a compound which has been disclosed in JP-A-51-138642. The results are shown in Table 4. With regard to histological observation, each of the rat groups of 10, 50 and 100 mg doses showed no evident appearance of vacuoles inside the acinic cells, and the appearance of interstitial edema was almost negligible. These changes became significant as the oral dose of Compound 39 increased, thus confirming the effect of Compound 39 to prevent pancreatitis.

In the Comparative Example 1, the rat group of 100 mg oral administration showed decrease in the pancreatitis-specific signs, but the reducing effects were inferior to those of Compound 39.

TABLE 4

| RESULTS OF EVALUATION | | |
|---|---|---|
| | % Pancreas wt. | Serum amylase (IU/ml) |
| No treatment | 0.38 ± 0.06 | 134 ± 11 |
| Administration of caerulein | 0.88 ± 0.07 | 817 ± 103 |
| Plus Compound 39 10 mg/kg | 0.99 ± 0.16 | 556 ± 31 |
| Plus Compound 39 50 mg/kg | 0.66 ± 0.04 | 362 ± 15 |
| Plus Compound 39 100 mg/kg | 0.39 ± 0.06 | 99 ± 17 |
| Plus Comparative Compound 100 mg/kg | 0.47 ± 0.09 | 216 ± 7 |

## INDUSTRIAL APPLICABILITY

Since the compound of the present invention represented by the general formula (I) and its pharmacologically acceptable acid addition salts are possessed of strong activities to inhibit various serine proteases such as trypsin, thrombin, plasmin, kallikrein, factor Xa, elastase and the like, they are applicable for example to an anti-trypsin agent which is useful for the treatment of pancreatitis, an anti-plasmin agent useful for the treatment of hemorrhagic diseases and an anti-thrombin agent useful for the treatment of thrombosis. They are also useful in new application fields as an elastase inhibitor.

While the invention has been described in detail and with reference to specific examples thereof, it will be apparent to one skilled in the art that various changes and modifications can be made therein without departing from the spirit and scope thereof.

## Claims

**1.** A 4-guanidinobenzoic acid phenyl ester derivative represented by the following general formula (I) or a pharmacologically acceptable acid addition salt thereof;

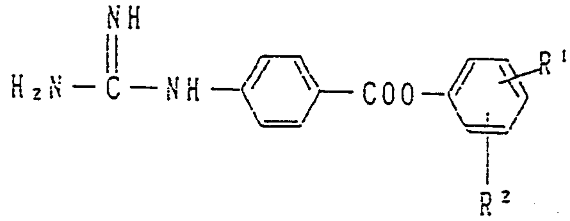

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group.

**2.** A proteolytic enzyme inhibitor which contains a 4-guanidinobenzoic acid phenyl ester derivative represented by the following general formula (I) or a pharmacologically acceptable acid addition salt thereof as the active ingredient;

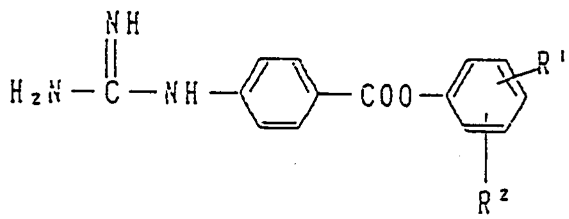

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group.

**3.** A process for producing 4-guanidinobenzoic acid phenyl ester derivative by the following general formula (I):

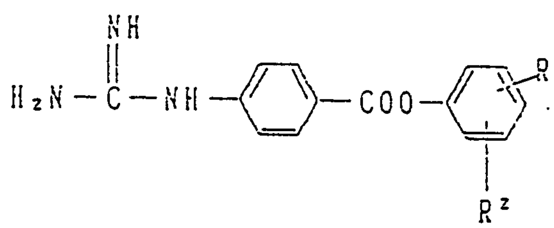

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group,

which comprises allowing a 4-guanidinobenzoic acid represented by the following general formula (II) to undergo condensation reaction with a compound represented by the following general formula (III) in the absence of solvent or in an organic solvent in the presence of a dehydrating agent;

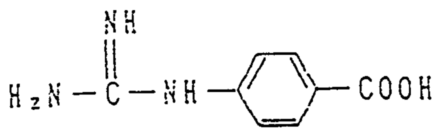

(II)

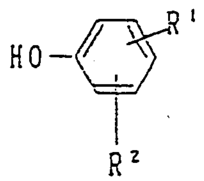

(III)

wherein $R^1$ and $R^2$ are the same as those described in the foregoing.

**4.** A process for producing 4-guanidinobenzoic acid phenyl ester derivative represented by the following general formula (I):

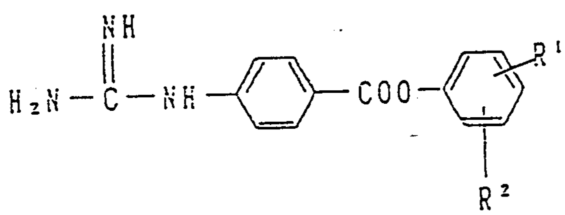

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group,

which comprises allowing a compound represented by the following general formula (IV) to react with a compound represented by the following general formula (III) in an aqueous solvent or in an organic solvent in the presence of an acid bonding agent;

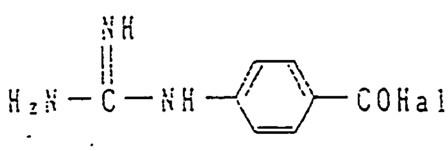

(IV)

wherein Hal is chlorine atom, bromine atom or iodine atom;

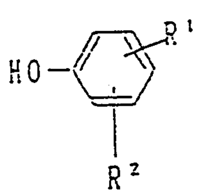

(III)

wherein $R^1$ and $R^2$ are the same as those described in the foregoing.

**Amended claims**

**1.** (amended) A 4-guanidinobenzoic acid phenyl ester derivative represented by the following general formula (I) or a pharmacologically acceptable acid addition salt thereof;

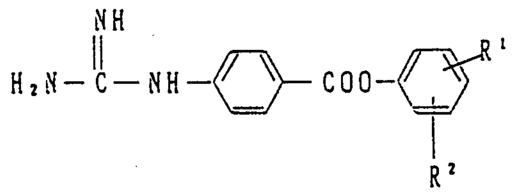

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group, with the proviso that when $R^1$ is a hydrogen atom, $R^2$ is not a group represented by $OCOR^3$.

**2.** (amended) A proteolytic enzyme inhibitor which contains a 4-guanidinobenzoic acid phenyl ester derivative represented by the following general formula (I) or a pharmacologically acceptable acid addition salt thereof as the active ingredient;

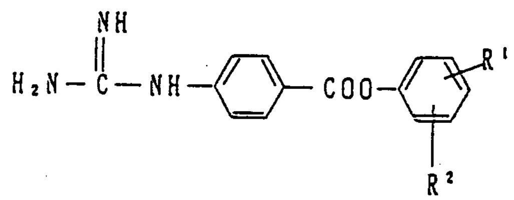

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group, with the proviso that when $R^1$ is a hydrogen atom, $R^2$ is not a group represented by $OCOR^3$.

**3.** (amended) A process for producing 4-guanidinobenzoic acid phenyl ester derivative by the following general formula (I):

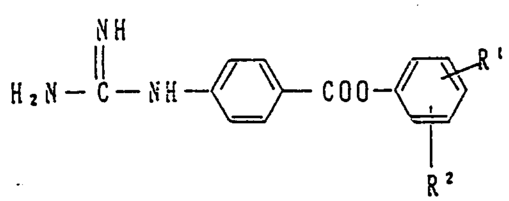

(I)

wherein $R^1$ is a hydrogen atom or halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group, with the proviso that when $R^1$ is a hydrogen atom, $R^2$ is not a group represented by $OCOR^3$,

which comprises allowing a 4-guanidinobenzoic acid represented by the following general formula (II) to undergo condensation reaction with a compound represented by the following general formula (III) in the absence of solvent or in an organic solvent in the presence of a dehydrating agent;

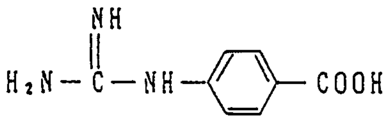

(II)

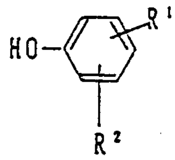

(III)

wherein $R^1$ and $R^2$ are the same as those described in the foregoing.

**4.** (amended) A process for producing 4-guanidinobenzoic acid phenyl ester derivative represented by the following general formula (I):

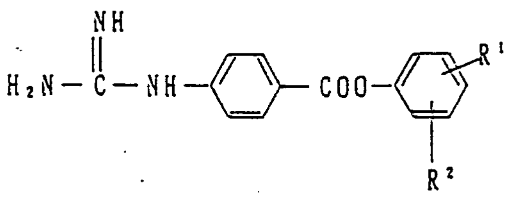

(I)

wherein $R^1$ is a hydrogen atom or a halogen atom; $R^2$ is a trifluoromethoxy group, a group represented by $OCOR^3$, a group represented by $C(=NOR^4)R^5$ or a styrene sulfonyl group; $R^3$ is a substituted or unsubstituted $C_{3-7}$ cycloalkyl group; $R^4$ is a hydrogen atom, a $C_{1-4}$ alkyl group, a $C_{1-4}$ alkenyl group or a group represented by $CH_2COOR^6$; $R^5$ is a hydrogen atom or a $C_{1-4}$ alkyl group; and $R^6$ is a hydrogen atom or a $C_{1-4}$ alkyl group, with the proviso that when $R^1$ is a hydrogen atom, $R^2$ is not a group represented by $OCOR^3$,

which comprises allowing a compound represented by the following general formula (IV) to react with a compound represented by the following general formula (III) in an aqueous solvent or in an organic solvent in the presence of an acid bonding agent;

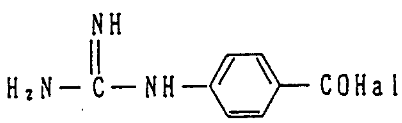

(IV)

wherein Hal is chlorine atom, bromine atom or iodine atom;

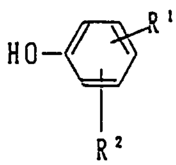

(III)

wherein $R^1$ and $R^2$ are the same as those described in the foregoing.

# INTERNATIONAL SEARCH REPORT

International Application No PCT/JP91/00773

**I. CLASSIFICATION OF SUBJECT MATTER** (if several classification symbols apply, indicate all) [6]

According to International Patent Classification (IPC) or to both National Classification and IPC

Int. $Cl^5$ C07C279/18

**II. FIELDS SEARCHED**

Minimum Documentation Searched [7]

| Classification System | Classification Symbols |
|---|---|
| IPC | C07C279/18, C07C277/08, A61K31/245 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 55-100356 (Kowa Co., Ltd.), July 31, 1980 (31. 07. 80), Claims 1 to 5, line 1, lower right column, page 3 to line 15, lower right column, page 4 (Family: none) | 1-4 |
| Y | JP, A, 53-147044 (Ono Pharmaceutical Co., Ltd.), December 21, 1978 (21. 12. 78), Claims 1 to 4, 2nd line from the bottom, upper right column to line 1, lower right column, page 3 (Family: none) | 1-4 |
| Y | JP, A, 63-165357 (Ono Pharmaceutical Co., Ltd.), July 8, 1988 (08. 07. 88), Claims 1 to 2 & EP, A, 222608 | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 30, 1991 (30. 08. 91) | September 17, 1991 (17. 09. 91) |
| **International Searching Authority** | **Signature of Authorized Officer** |
| Japanese Patent Office | |